# FASCICULE DE BREVET EUROPEEN

(11) **EP 2 000 116 B1**
(45) Date de publication et mention de la délivrance du brevet: **08.08.2012**
(21) Numéro de dépôt: 08104298.8
(22) Date de dépôt: 06.06.2008
(51) Int. Cl.: A61F 2/24

(54) **Nécessaire destiné à être implanté dans un conduit de circulation de sang**
Kit zum Implantieren in eine Blutkreislaufbahn
Kit designed to be implanted in a blood vessel

(30) Priorité: 08.06.2007 FR 0755603
(43) Date de publication de la demande: 10.12.2008
(73) Titulaire: Laboratoires Perouse, 60173 Ivry Le Temple (FR)
(72) Inventeur: STYRC, Mikolaj, L-8190, KOPSTAL (LU)
(74) Mandataire: Domenego, Bertrand

(56) Documents cités:
- WO-A-00/47139
- WO-A-2005/070343
- WO-A-2007/009609
- FR-A- 2 883 721
- US-A1- 2002 032 481
- US-A1- 2005 004 665

## Description

La présente invention concerne un nécessaire destiné à être implanté dans un conduit de circulation du sang, du type comprenant :
- une endoprothèse tubulaire présentant une surface intérieure délimitant un canal d'axe longitudinal ; et
- une valve prothétique mobile par rapport à l'endoprothèse tubulaire pour être implantée dans le canal, la valve prothétique comprenant :
   - une armature porteuse présentant une surface extérieure destinée à être appliquée contre la surface intérieure de l'endoprothèse, l'armature étant déformable radialement depuis une position repliée de mise en place vers une position déployée d'implantation ;
   - un obturateur souple, lié à l'armature, et déformable entre une position d'obstruction dans laquelle il est étendu transversalement et une position de libération dans laquelle il est contracté transversalement sous l'action du flux circulant dans le canal ;

l'endoprothèse comprenant au moins une butée de calage de la valve prothétique dans le canal pour bloquer le déplacement axial de la surface extérieure le long de la surface intérieure suivant au moins un premier sens.

On connaît de EP-A-850 607 un nécessaire comprenant une endoprothèse tubulaire et une valve prothétique qui présente une armature porteuse déformable et un obturateur souple fixé sur l'armature.

Un tel nécessaire est destiné à être implanté en remplacement d'une valve, dans un conduit de circulation du sang.

De telles valves sont par exemple présentes dans le coeur, entre les oreillettes et les ventricules, ou à la sortie du ventricule droit et du ventricule gauche. Ces valves assurent une circulation univoque du flux sanguin, évitant un reflux sanguin à l'issue de la contraction ventriculaire.

Pour effectuer un remplacement de valve, l'endoprothèse tubulaire prévue dans le nécessaire est implantée dans la partie du conduit dans lequel se trouve la valve malade. Puis, la valve prothétique dans son état replié est amenée dans le canal intérieur délimité par l'endoprothèse et est appliquée contre cette endoprothèse par gonflage d'un ballon.

Un tel dispositif ne donne pas entière satisfaction. En effet, le positionnement relatif de la valve prothétique par rapport à l'endoprothèse est approximatif, et la fixation de la valve dans l'endoprothèse est peu robuste.

Pour pallier ce problème, on connaît de FR-A-2 883 721, un nécessaire du type précité, dans lequel des butées de calage de la valve prothétique sont formées par déformation de l'armature porteuse, afin de réaliser des tronçons de section transversale variable le long de l'axe longitudinal. La surface extérieure de l'armature porteuse est ainsi calée entre une butée distale et une butée proximale lors de l'implantation de la valve dans l'endoprothèse.

Un tel nécessaire ne donne pas entière satisfaction. En effet, l'armature tubulaire de l'endoprothèse avec des butées peut être difficile à réaliser. En outre, la présence de butées transversales formées dans l'armature augmente l'encombrement radial de l'endoprothèse lors de son introduction dans le corps du patient, puisque les butées limitent la compression radiale de l'endoprothèse dans son outil d'introduction.

Un but de l'invention est donc de fournir un nécessaire du type précité, qui permette l'implantation précise et robuste d'une valve prothétique dans une armature tubulaire préalablement implantée, sans augmenter l'encombrement radial de l'armature lors de son introduction dans le corps du patient.

A cet effet, l'invention a pour objet un nécessaire du type précité, caractérisé en ce que la butée comprend au moins un lien souple s'étendant transversalement à travers le canal entre deux points de liaison situés sur la surface intérieure et espacés angulairement autour de l'axe longitudinal.

Le nécessaire selon l'invention peut comprendre l'une ou plusieurs des caractéristiques suivantes, prise(s) isolément ou suivant toute(s) combinaison(s) techniquement possible(s) :
- l'endoprothèse tubulaire est déployable entre un état comprimé et un état dilaté, le ou chaque lien souple étant déployable conjointement avec l'endoprothèse entre une configuration distendue lorsque l'endoprothèse occupe son état comprimé et une position tendue à travers le canal lorsque l'endoprothèse occupe son état dilaté ;
- le ou chaque lien souple est libre par rapport à la surface intérieure entre ses points de liaison ;
- les points de liaison du ou de chaque lien souple s'étendent sensiblement dans un même plan perpendiculaire à l'axe longitudinal ;
- la ou chaque butée comprend au moins deux liens souples s'étendant chacun entre deux points de liaison, les points de liaison formant les sommets d'un polygone ;
- l'endoprothèse comprend une butée proximale et une butée distale espacées axialement, chaque butée comprenant au moins un lien souple s'étendant transversalement à travers le canal entre deux points de liaison situés sur la surface intérieure et espacés angulairement autour de l'axe longitudinal ;
- le ou chaque lien d'au moins une butée délimite, un passage central dans le canal, l'armature porteuse de la valve prothétique dans sa position repliée de mise en place étant apte à être introduite entre la butée distale et la butée proximale à travers le passage central ;
- la distance séparant axialement la butée proximale de la butée distale est supérieure ou sensiblement égale à la longueur de la surface extérieure placée en contact avec la surface intérieure, prise le long de l'axe longitudinal ; et
- le ou chaque lien souple est formé par un fil.

L'invention sera mieux comprise à la lecture de la description qui va suivre, donnée uniquement à titre d'exemple, et faite en se référant aux dessins annexés, sur lesquels :
- la Figure 1 est une vue schématique partielle en perspective d'un premier nécessaire selon l'invention, avant son implantation dans un vaisseau sanguin ;
- la Figure 2 est une vue en coupe suivant un plan axial médian du premier nécessaire selon l'invention, implanté dans un conduit de circulation du sang ;
- la Figure 3 est une vue analogue à la Figure 2 lors de l'implantation de la valve prothétique dans l'endoprothèse ;
- la Figure 4 est une vue en section suivant un plan transversal d'un deuxième nécessaire selon l'invention.
- les Figures 5 à 7 sont des vues analogues à la Figure 4 de variantes du deuxième nécessaire ; et
- la Figure 8 est une vue analogue à la Figure 2 d'un troisième nécessaire selon l'invention muni d'une valve interchangeable ; et

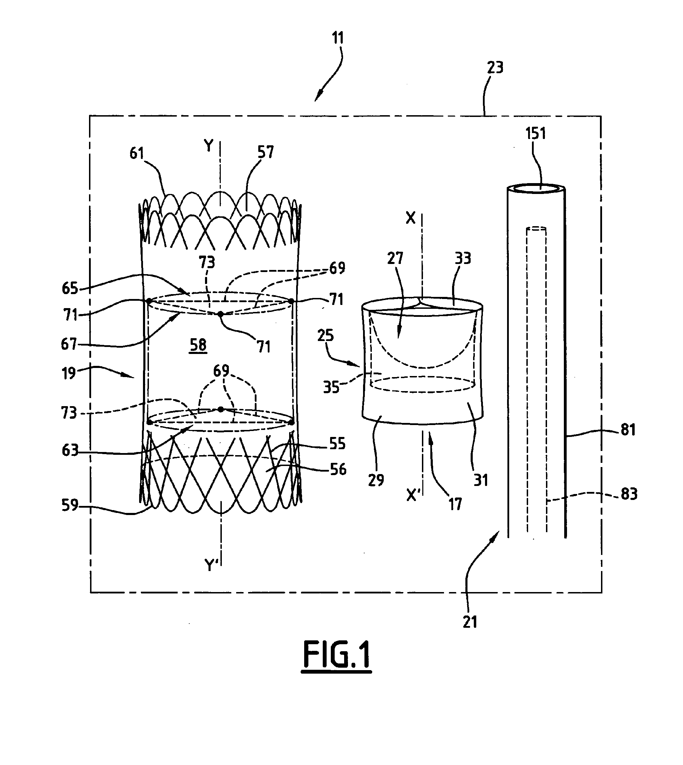

Un premier nécessaire 11 selon l'invention est représenté sur les Figures 1 à 3.

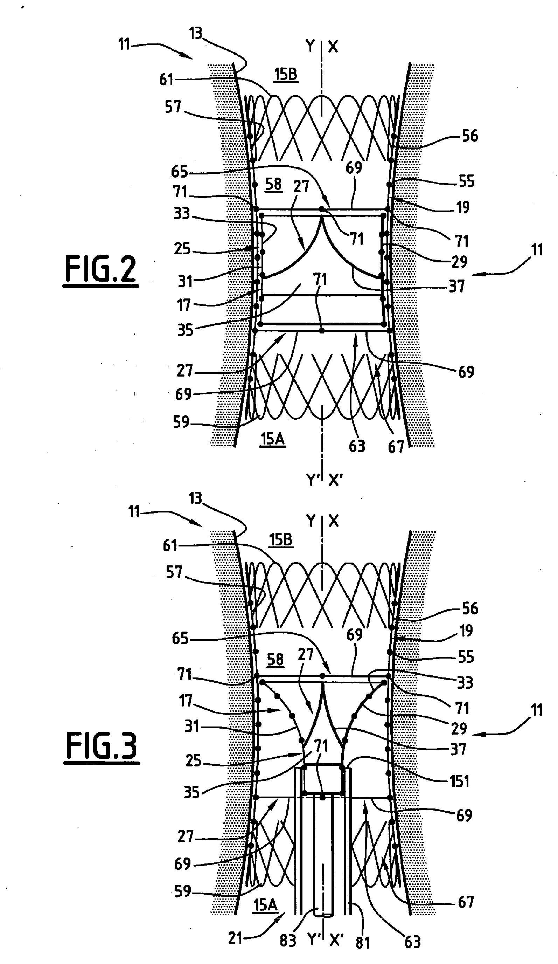

La Figure 1 représente le nécessaire 11 avant son implantation dans un conduit de circulation du sang, alors que la Figure 2 représente le nécessaire 11 implanté dans un conduit 13 de circulation du sang. Le conduit 13 est par exemple une artère pulmonaire reliée à son extrémité proximale 15A à la sortie du ventricule droit du coeur, notamment d'un être humain, et à son extrémité distale 15B au poumon.

Comme illustré par la Figure 1, le nécessaire 11 comprend une valve prothétique 17, une endoprothèse 19 destinée à recevoir la valve prothétique 17, et un outil 21 d'implantation de la valve prothétique 17 dans l'endoprothèse 19.

Le nécessaire 11 est par exemple conservé dans un emballage 23.

Comme illustré par la Figure 2, la valve prothétique 17 comporte une armature porteuse 25 et un obturateur déformable 27, supporté par l'armature 25 et solidaire de celle-ci.

La valve 17 est mobile par rapport à l'endoprothèse 19, lors de sa mise en place dans l'endoprothèse 19, entre une position initiale de stockage à l'écart de l'endoprothèse 19, représentée sur la Figure 1, et une position montée dans l'endoprothèse 19, représentée sur la Figure 2.

L'armature 25 est formée par un treillis tubulaire 29 auto-expansible d'axe X-X', déployable spontanément entre une configuration repliée de mise en place, radialement de petit diamètre, et une configuration déployée radialement de grand diamètre. Le treillis tubulaire 29 délimite une surface périphérique extérieure 31 appliquée contre l'endoprothèse 19 lorsque la valve 17 est montée dans celle-ci, et une surface périphérique intérieure 33 sur laquelle est fixé l'obturateur 27.

L'obturateur 27 est par exemple réalisé à base d'une valve native d'un animal bovin ou ovin. En variante, il est réalisé à base de tissu naturel ou synthétique.

L'obturateur 27 comprend une base tubulaire 35 fixée sur la surface intérieure 33 et prolongée vers le haut par trois feuillets d'obturation 37.

Les feuillets 37 sont venus de matière avec la base 35. Ils sont répartis autour de l'axe X-X'. Les feuillets 37 sont déformables entre une configuration d'obstruction, dans laquelle ils obturent sensiblement la lumière intérieure 44 et une configuration de libération, dans laquelle ils sont plaqués contre le treillis tubulaire 29 pour permettre le passage du sang à travers la valve 17.

L'endoprothèse 19 est formée d'un treillis tubulaire d'axe central Y-Y' de fils entrelacés 55, noyés dans un film 56 extensible et étanche aux liquides tel qu'un élastomère.

Le treillis 55 est obtenu par tressage d'au moins un fil d'acier inoxydable, d'un alliage à mémoire de forme, ou d'un polymère. En variante, le treillis 55 est obtenu par découpe laser d'un tube.

Comme connu en soi, le treillis 55 de l'endoprothèse est susceptible de se déformer spontanément d'un état comprimé, dans lequel il présente un petit diamètre, vers un état dilaté, dans lequel il présente un diamètre supérieur, cet état dilaté constituant son état de repos.

Dans l'état implanté, tel qu'illustré sur la Figure 2, l'endoprothèse 19 s'applique, du fait de son élasticité, contre la surface interne du conduit 13, constituant ainsi une gaine intérieure au conduit 13.

L'endoprothèse 19 présente une surface intérieure 57 qui délimite un canal 58 de circulation du flux sanguin, d'axe Y-Y', débouchant à l'extrémité proximale 59 et à l'extrémité distale 61 de l'endoprothèse 19

La surface intérieure 57 de l'endoprothèse est globalement continûment dérivable, de sorte que le canal 58 délimite une section transversale variant de manière continue depuis l'extrémité proximale 59 vers l'extrémité distale 61 de l'endoprothèse 39.

L'endoprothèse 19 comprend une butée proximale 63 et une butée distale 65 de calage de la valve 17, pour empêcher le déplacement axial de la valve 17 respectivement vers l'extrémité proximale 59 et vers l'extrémité distale 61 du treillis tubulaire 55.

Comme illustré par les Figures 1 à 3, chaque butée 63, 65 est formée par une structure transversale 67 de liens filiformes en forme de polygone, déployable dans le canal 58 de circulation du flux sanguin.

La butée proximale 63 est décalée axialement par rapport à la butée distale 65. La distance qui sépare les butées 63, 65, prise le long de l'axe Y-Y' est supérieure ou sensiblement égale à la longueur de la surface extérieure 31 du treillis tubulaire 29, comme illustré sur la Figure 2.

Dans l'exemple représenté par la Figure 1, chaque butée 63, 65 est formée par trois liens filiformes 69 en forme de triangle, déployables entre une configuration distendue lors de l'introduction de l'endoprothèse 19 dans le corps humain, et une configuration tendue après déploiement de l'endoprothèse 19 dans son état dilaté.

Dans cet exemple, chaque lien filiforme 69 est constitué d'un fil fixé à ses extrémités sur la surface intérieure 57 de l'endoprothèse 19 en deux points de liaison 71, par l'intermédiaire d'attaches. Chaque lien filiforme 69 est par ailleurs libre par rapport à la surface intérieure 57 entre ses extrémités. Les points de liaison 71 des liens filiformes 69 sont répartis angulairement autour de l'axe Y-Y' de l'endoprothèse 19 et sont situés sensiblement dans un même plan transversal par rapport à l'axe Y-Y'.

La structure de liens filiformes 67 s'étend dans un plan transversal lorsque les liens filiformes 69 sont déployés dans le canal 58. Les points de liaison 71 forment ainsi les sommets d'un polygone dont les cotés sont délimités par les liens 67.

Dans le canal 58, les liens filiformes 69 délimitent entre eux un passage central 73 de circulation dégagé pour l'introduction des moyens d'implantation 21 de la valve 17 dans le conduit 58, comme on le verra plus bas.

Les liens filiformes 69 sont formés par exemple d'acier inoxydable, d'un polymère ou d'un métal à mémoire de forme. Ils présentent un diamètre faible, inférieur à au moins dix fois le diamètre du canal 58 lorsque l'endoprothèse 19 occupe son état dilaté.

L'outil d'implantation 21 comprend un cathéter 81 de maintien de la valve 17 dans sa configuration rétractée et un tuteur 83 de poussée de la valve 19 à insérer dans le cathéter 81.

Le cathéter 81 présente une section transversale intérieure inférieure à la section transversale de la valve 17 dans sa configuration déployée, et une section transversale extérieure inférieure à la section intérieure de l'endoprothèse 19 et à celle du passage central 73 délimité entre les liens filiformes 69 de la butée proximale 63 et de la butée distale 65.

Le tuteur 83 est déplaçable axialement à l'intérieur du cathéter 81. II porte à son extrémité distale la valve 17 avant son implantation dans l'endoprothèse 19.

Le fonctionnement du premier nécessaire 10 selon l'invention va maintenant être décrit. Initialement, le chirurgien charge la valve 17 dans son outil d'implantation 21. A cet effet, il fixe la valve 17 par rapport au tuteur 83 et rétracte radialement la valve 17 pour la faire pénétrer dans le cathéter 81, comme connu en soi.

L'armature porteuse 25 de la valve 17 est alors comprimée entre le tuteur 83 et le cathéter 81 dans sa configuration de faible diamètre, en vue de son introduction dans le corps humain.

De même, le chirurgien contracte l'endoprothèse 19 pour qu'elle occupe son état comprimé dans un outil d'introduction (non représenté). Lorsque l'endoprothèse 19 occupe son état comprimé, les liens filiformes 69 des butées 63, 65 sont distendus dans le canal central 58. Les liens filiformes 69 occupant un volume très faible dans le canal, et la surface intérieure du treillis 55 étant dépourvue de discontinuités de grande amplitude, il est possible de contracter radialement l'endoprothèse 19 pour diminuer fortement son encombrement lors de l'introduction dans le patient.

De manière connue, le chirurgien introduit alors par voie percutanée l'endoprothèse 19 dans le corps du patient, jusqu'à sa position d'implantation dans le conduit 13. Puis, il procède au largage de l'endoprothèse 19 dans le conduit par expansion radiale vers son état dilaté.

Le treillis 55 de l'endoprothèse se plaque alors contre la paroi du conduit 13.

Lors de ce déploiement, les liens filiformes 69 des butées 63, 65 se tendent. Les structures 67 de liens filiformes en forme de polygone sont alors déployées à travers le canal 68 pour former les butées 63, 65. Chaque lien 69 s'étend alors chacune linéairement, sensiblement dans un plan perpendiculaire à l'axe Y-Y' dans le canal 58 à l'écart de la surface intérieure 57, à l'exception des points de liaison 71.

Comme illustré par la Figure 3, l'outil 21 est alors introduit dans le corps du patient par voie percutanée, en maintenant la valve 17 contractée entre le tuteur 83 et le cathéter 81. L'outil 21 est alors placé de sorte que son extrémité distale soit introduite dans le canal intérieur 58 de l'endoprothèse 19, entre les butées 63, 65, à travers le passage central 73 délimité par les liens filiformes 69 de la butée proximale 63.

Ceci étant fait, le cathéter 81 est déplacé de manière proximale par rapport au tuteur 83, lequel est maintenu en position entre les butées 63, 65. Le déplacement du cathéter 81 découvre progressivement l'armature porteuse 25 de la valve 17 et provoque l'expansion radiale de son bord distal dans le canal 58. Le bord distal de l'armature 25 présente alors un diamètre extérieur supérieur à la dimension transversale maximale du passage 73 délimité entre les liens filiformes 69 de la butée distale 65. Le chirurgien déplace alors la valve 17 par l'intermédiaire du tuteur 83 jusqu'à ce que le bord distal de l'armature 25 bute contre les liens filiformes 69.

Puis, il découvre totalement l'armature 25 et relâche alors le bord proximal de la valve 17. La surface extérieure 31 de l'armature se cale contre la surface intérieure 57 de l'endoprothèse 19. Par ailleurs, l'armature 25 est calée axialement entre la butée proximale 63, qui empêche son déplacement dans un premier sens vers l'extrémité proximale 59 de l'endoprothèse 19, et la butée distale 65, qui empêche son déplacement dans un deuxième sens vers l'extrémité distale 61 de l'endoprothèse 19.

La valve 19 est donc positionnée de manière très précise dans l'endoprothèse 19. Elle est maintenue en position axiale de manière robuste et durable par les butées 63, 65 réalisées par des liens filiformes. L'axe X-X' du treillis 23 est sensiblement coaxial avec l'axe Y-Y' du treillis 55.

La faible surface occupée par les butées 67, 69 évite en outre de diminuer sensiblement le flux de sang à travers le nécessaire 11. En outre, les butées 63, 65 sont réalisées de manière simple et peu coûteuse, sans avoir à modifier la structure du treillis de l'endoprothèse 19.

La valve prothétique 17 implantée fonctionne de la manière suivante. A l'issue d'une expulsion du ventricule droit, lorsque celui-ci augmente de volume, le flux sanguin est aspiré dans le conduit 13, depuis l'extrémité distale 15B vers l'extrémité proximale 15A. Le sang emplit alors les feuillets 37, lesquels se déplacent radialement vers l'axe X-X' obturant ainsi de manière sensiblement étanche le conduit 13.

Lors d'une contraction du ventricule droit, le sang circule de l'extrémité proximale 15A vers l'extrémité distale 15B. Les feuillets 37 sont alors plaqués contre la surface intérieure 33 de l'armature porteuse 25, ce qui provoque l'ouverture du canal 58. Le flux sanguin est alors libre de circuler dans le conduit 13 de part et d'autre de la valve 17.

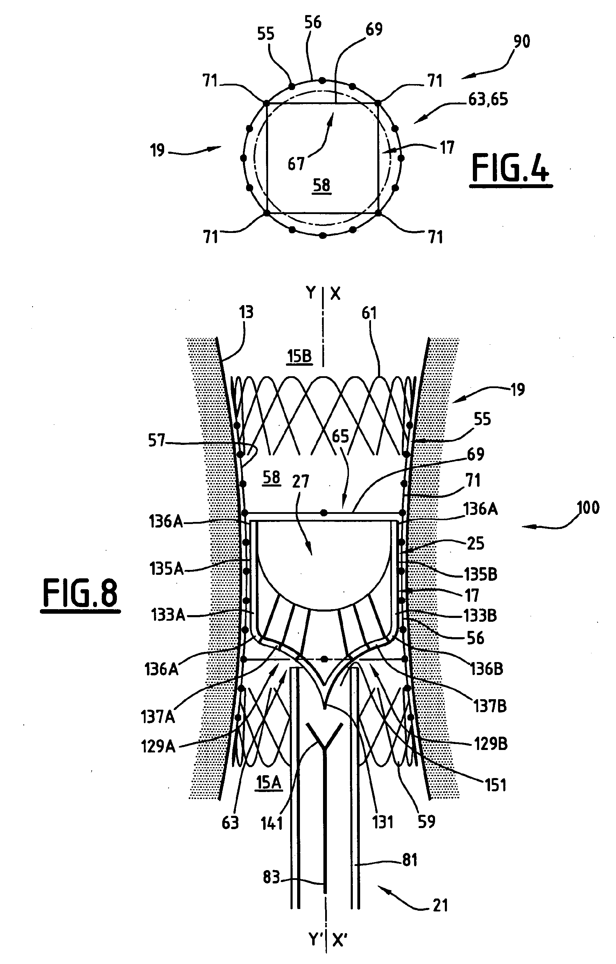

Dans le deuxième nécessaire 90 selon l'invention, représenté sur la Figure 4, chaque butée 63, 64 est formée par un polygone comprenant au moins quatre côtés.

Dans une autre variante, la structure polygonale 67 est formée par un fil unique fermé engagé, à travers le treillis au niveau des points de liaison 71. Le fil unique délimite un polygone fermé et occupe une position globalement invariante par rapport à l'endoprothèse 19.

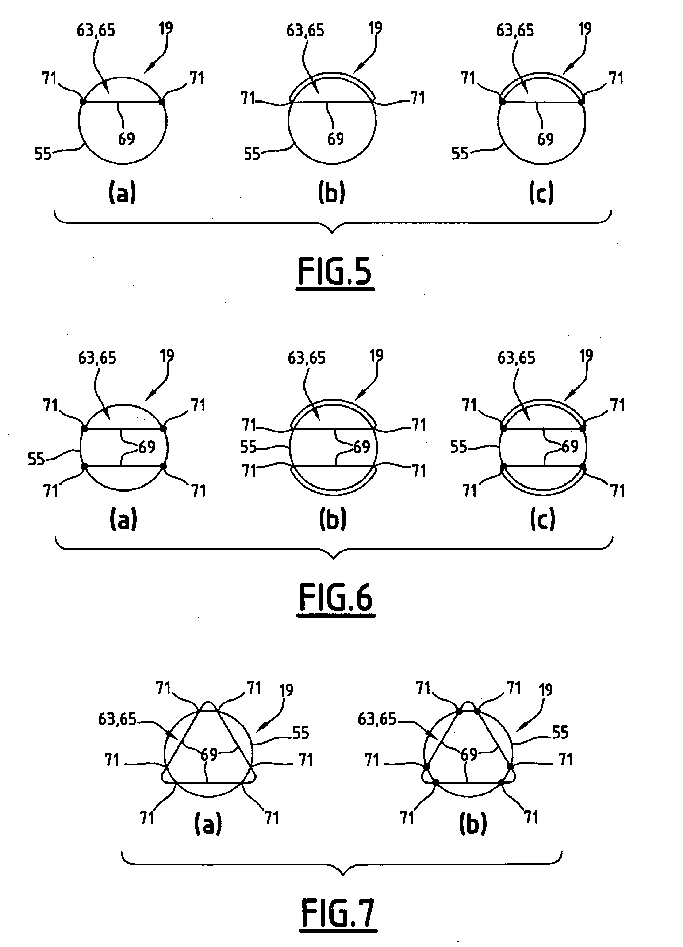

La Figure 5 illustre des variantes dans lesquelles chaque butée 63, 65 comprend un lien filiforme unique 69 s'étendant transversalement dans le canal 58. Ce lien 69 est réalisé à partir d'un fil unique. Dans la configuration de la Figure 5(a), le fil unique est fixé à ses extrémités sur le treillis 55, au niveau des points de liaison 71 sur la surface intérieure 57. Dans la configuration des Figure 5(b) et 5(c), le fil unique est enroulé en boucle pour délimiter le lien filiforme 69 placé dans le canal 58 et un tronçon extérieur enroulé autour et à l'extérieur du treillis 55 entre les points de liaison 71. Dans configuration de la Figure 5(c), le fil est en outre fixé par des attaches sur le treillis 55, alors qu'il est librement engagé à travers le treillis 55 dans la configuration de la Figure 5(b).

La Figure 6 illustre des variantes de la Figure 5, dans lesquelles chaque butée 63, 65 est formée par deux liens filiformes 69 disposés parallèlement l'un à l'autre dans le canal 58. Chaque lien 69 est formé par un fil suivant les configurations illustrées par la Figure 5.

La Figure 7 illustre deux variantes de la Figure 1 dans lesquelles la structure 67 avec trois liens 69 en forme de triangle est réalisée à base d'un fil unique engagé à travers le treillis au niveau des sommets du triangle. La structure 67 comprend ainsi deux points de liaison 71 au niveau de chaque sommet.

Dans le troisième nécessaire 100 selon l'invention représenté sur la Figure 8, la valve 17 est une valve prothétique interchangeable. A cet effet, elle comporte des moyens intégrés pour sa compression centripète en vue de sa récupération après implantation.

Plus précisément, l'armature 25 est formée d'au moins deux branches 129A, 129B et notamment de trois branches reliées l'une à l'autre à une première extrémité 131 pour former une pince élastiquement déformable entre une position déployée, dans laquelle les branches sont écartées de l'axe médian X-X', et une position repliée, dans laquelle les deux branches 129A, 129B sont rapprochées de l'axe médian X-X'.

Les deux branches 129A, 129B sont généralement symétriques par rapport à l'axe médian X-X' confondu avec l'axe du conduit, après implantation.

La longueur des branches, mesurée suivant l'axe X-X', est comprise entre 2 et 4 cm et est de préférence égale à 3 cm.

Chaque branche 129A, 129B comporte un tronçon 133A, 133B d'appui sur l'endoprothèse 19. Chaque tronçon d'appui 133A, 133B est constitué d'un segment rectiligne s'étendant généralement suivant une génératrice de l'endoprothèse 19, lorsque l'armature 25 est déployée. Chaque tronçon d'appui 133A, 133B présente une surface extérieure 135A, 135B en appui sur l'endoprothèse 19.

La longueur des tronçons d'appui entre un bord proximal 136A et un bord distal 136B est de 1 à 3 cm et de préférence d'environ 2 cm.

Cette longueur est sensiblement égale à la distance séparant les butées 63, 65.

Ces tronçons d'appui 133A, 133B sont prolongés par des tronçons de manoeuvre 137A, 137B convergents l'un vers l'autre jusqu'au point de liaison 31. Les tronçons de manoeuvre sont généralement inclinés par rapport à l'axe médian X-X'.

Les tronçons de manoeuvre 137A, 137B sont généralement courbes et présentent un centre de courbure disposé hors de l'espace délimité entre les deux branches. Ainsi, les tronçons 137A, 137B sont bombés vers l'intérieur de la pince.

Dans ce troisième nécessaire 100, l'outil 21 de mise en place de la valve 17 est également un outil de retrait de cette valve. A cet effet, le tuteur de traction 83 comprend une mâchoire 141 à son extrémité distale.

La mise en place et le fonctionnement du troisième nécessaire 100 selon l'invention est analogue à celui du premier nécessaire 10.

Toutefois, la valve prothétique 17 étant équipée de moyens de compression centripète, elle peut être ramenée dans son état comprimé et évacuée de manière transluminale.

Plus précisément, pour le retrait de la valve prothétique 17, le cathéter 81 est introduit au travers de l'oreillette droite et du ventricule droit et est disposé en regard de l'extrémité 131 de l'armature porteuse en forme de pince.

Le tuteur de traction 83 est acheminé au travers du cathéter 81. La mâchoire 141 saisit alors l'extrémité 31 de la pince. Alors que l'extrémité ouverte, notée 151, du cathéter 81 est en contact avec les tronçons de manoeuvre 137A, 137B, l'armature porteuse est progressivement tirée à l'intérieur du cathéter 81. Par effet de came, les deux bras 129A, 129B sont resserrés l'un vers l'autre et la valve prothétique est progressivement amenée dans son état resserré et introduite dans le cathéter 81. Le cathéter 81 renfermant la valve prothétique est alors extrait du corps humain.

Un nouveau cathéter contenant une nouvelle valve prothétique 17 est alors introduit dans le corps humain et la valve est larguée en procédant aux opérations exposées précédemment, en sens inverse.

En variante, les moyens de compression centripète de la valve 17 comprennent un trépied comme décrit dans la demande de brevet FR-A-2 874 812 ou un cordon périphérique comme décrit dans la demande de brevet FR-A-2 847 800.

## Revendications

1. Nécessaire (11 ; 90 ; 100) destiné à être implanté dans un conduit (13) de circulation du sang, du type comprenant :
- une endoprothèse tubulaire (19) présentant une surface intérieure (57) délimitant un canal (58) d'axe longitudinal (Y-Y') ; et
- une valve prothétique (17) mobile par rapport à l'endoprothèse tubulaire (19) pour être implantée dans le canal (58), la valve prothétique (17) comprenant :
• une armature porteuse (25) présentant une surface extérieure (31) destinée à être appliquée contre la surface intérieure (57) de l'endoprothèse, l'armature (25) étant déformable radialement depuis une position repliée de mise en place vers une position déployée d'implantation ;
• un obturateur souple (27), lié à l'armature (25), et déformable entre une position d'obstruction dans laquelle il est étendu transversalement et une position de libération dans laquelle il est contracté transversalement sous l'action du flux circulant dans le canal (58) ;
l'endoprothèse (19) comprenant au moins une butée (63, 65) de calage de la valve prothétique (17) dans le canal (58) pour bloquer le déplacement axial de la surface extérieure (31) le long de la surface intérieure (57) suivant au moins un premier sens ;
**caractérisé en ce que** la butée (63, 65) comprend au moins un lien souple (69) s'étendant transversalement à travers le canal (58) entre deux points de liaison (71) situés sur la surface intérieure (57) et espacés angulairement autour de l'axe longitudinal (Y-Y').

2. Nécessaire (11 ; 90 ; 100) selon la revendication 1, **caractérisé en ce que** l'endoprothèse tubulaire (19) est déployable entre un état comprimé et un état dilaté, le ou chaque lien souple (69) étant déployable conjointement avec l'endoprothèse (19) entre une configuration distendue lorsque l'endoprothèse (19) occupe son état comprimé et une position tendue à travers le canal (58) lorsque l'endoprothèse (19) occupe son état dilaté.

3. Nécessaire (11 ; 90 ; 100) selon l'une des revendications 1 ou 2, **caractérisé en ce que** le ou chaque lien souple (69) est libre par rapport à la surface intérieure (57) entre ses points de liaison (71).

4. Nécessaire (11 ; 90 ; 100) selon l'une quelconque des revendications précédentes, **caractérisé en ce que** les points de liaison (71) du ou de chaque lien souple (69) s'étendent sensiblement dans un même plan perpendiculaire à l'axe longitudinal (Y-Y').

5. Nécessaire (11 ; 90 ; 100) selon l'une quelconque des revendications précédentes, **caractérisé en ce que** la ou chaque butée (63, 65) comprend au moins deux liens souples (69) s'étendant chacun entre deux points de liaison (71), les points de liaison formant les sommets d'un polygone.

6. Nécessaire (11 ; 90 ; 100) selon l'une quelconque des revendications précédentes, **caractérisé en ce que** l'endoprothèse (19) comprend une butée proximale (63) et une butée distale (65) espacées axialement, chaque butée (63, 65) comprenant au moins un lien souple (69) s'étendant transversalement à travers le canal (58) entre deux points de liaison (71) situés sur la surface intérieure (57) et espacés angulairement autour de l'axe longitudinal (Y-Y').

7. Nécessaire (11 ; 90 ; 100) selon la revendication 6, **caractérisé en ce que** le ou chaque lien (69) d'au moins une butée (63, 65) délimite, un passage central (73) dans le canal (58), l'armature porteuse (29) de la valve prothétique (17) dans sa position repliée de mise en place étant apte à être introduite entre la butée distale (65) et la butée proximale (63) à travers le passage central (73).

8. Nécessaire (11 ; 90 ; 100) selon l'une des revendications 6 ou 7, **caractérisé en ce que** la distance séparant axialement la butée proximale (63) de la butée distale (65) est supérieure ou sensiblement égale à la longueur de la surface extérieure (31) placée en contact avec la surface intérieure (57), prise le long de l'axe longitudinal (Y-Y').

9. Nécessaire (11 ; 90 ; 100) selon l'une quelconque des revendications précédentes, **caractérisé en ce que** le ou chaque lien souple (69) est formé par un fil.

## Claims

1. Kit (11; 90 ; 100) to be implanted in a blood circulation conduit (13), of the type comprising:
- a tubular endoprosthesis (19) having an inner surface (57) which delimits a channel (58) with a longitudinal axis (Y-Y'); and
- a prosthetic valve (17) which can be moved relative to the tubular endoprosthesis (19) in order to be implanted in the channel (58), the prosthetic valve (17) comprising:
• a supporting framework (25) having an outer surface (31) to be applied against the inner surface (57) of the endoprosthesis, the framework (25) being radially deformable from a folded fitting position to a deployed implantation position;
• a flexible obturator (27) which is connected to the framework (25), and is deformable between a closure position in which it is extended transversely, and a release position in which it is contracted transversely due to the flow circulating in the channel (58);
the endoprosthesis (19) comprising at least one stop (63, 65) for locking the prosthetic valve (17) in the channel (58) in order to block axial displacement of the outer surface (31) along the inner surface (57) in at least a first direction;
**characterized in that** the stop (63, 65) comprises at least one flexible connection (69) extending transversely across the channel (58) between two connection points (71) located on the inner surface (57) and spaced angularly about the longitudinal axis (Y - Y'),

2. Kit (11; 90 ; 100) according to claim 1, **characterized in that** the tubular endoprosthesis (19) can be deployed between a compressed state and a dilated state, the or each flexible connection (69) being deployable in conjunction with the endoprosthesis (15) between a slack configuration when the endoprosthesis (19) is in the compressed state and a tight position across the channel when the endoprosthesis (19) is in its dilated state.

3. Kit (11 ; 90 ; 100) according to one of claims 1 or 2, **characterized in that** the or each flexible connection (69) is free relative to the inner surface (57) between its connection point (71).

4. Kit (11 ; 90 ; 100) according to any one of the preceding claim **characterized in that** the connection points (71) of the or each flexible connection (69) extend substantially in the same plane perpendicular to the longitudinal axis (Y-Y').

5. Kit (11 ; 90 ; 100) according to any one of the preceding claims, **characterized in that** the or each stop (63, 65) comprises at least two flexible connections (69), each extending between two connection points (71), the connection points forming the vertices of polygon.

6. Kit (1) ; 90 ; 90) according to any one of the preceding claims, **characterized in that** the endoprosthesis (19) comprises a proximal stop (63) and a distal stop (65) which are at a distance from one another in the axial direction, each stop (63, 65) comprising at least a flexible connection (69) extending transversely across the channel (58) between two connection points (71) which are located on the inner surface (57) and are spaced angularly about the longitudinal axis (Y-Y').

7. Kit (11 ; 90 ; 100) according to claim 6, **characterized in that** the or each connection (69) of at least one stop (63, 65) delimit a central passage (73) in the channel (58), the supporting framework (29) of the prosthetic valve (17), in its folded fitting position, being able to be inserted between the distal stop (65) and the proximal stop (63) through the central passage (73).

8. Kit (11 ; 90 ; 100) according to one of claims 6 or 7, **characterized in that**, the axial distance separating the proximal stop (63) and the distal stop (65) is greater than or substantially equal to the length of the outer surface (31) placed in contact with the inner surface (57), taken along the longitudinal axis (Y-Y').

9. Kit (11 ; 90 ; 100) according to any one of the preceding claim, **characterized in that** the or each flexible connection (69) is formed by a thread.

## Patentansprüche

1. Kit (11; 90; 100) zum Implantieren in eine Blutheislaufbahn (13) des Typs umfassend:
- eine röhrenförmige Endoprothese (19), die ein innere Oberfläche (57) aufweist, die einen Kanal (58) mit einer Längsachse (Y-Y') begrenzt; und
- eine Klappenprothese (17), die mit Bezug auf die röhrenförmige Endoprothese (19) beweglich ist, um in den Kanal (58) implantiert zu werden, wobei die Klappenprothese (17) Folgendes umfasst:
• ein Tragegerüst (25), das eine äußere Oberfläche (31) aufweist, die ausgelegt ist, gegen die innere Oberfläche (57) der Endoprothese angebracht zu werden, wobei das Gerüst (25) radial am einer zusammengekiappten Position der Anbringung in eine ausgezogene Position der Implantation verformbar ist;
• eine flexible Verschussvorrichtung (27), die mit dem Gerüst (25) verbunden ist und zwischen einer Versehhussposition, in der sie quer liegt, und einer Freigabeposition, in der sie unter der Einwirkung des Flusses, der im Kanal (58) fließt, zusammengezogen ist, verformbar ist;
wobei die Endoprothese (19) mindestens einen Anschlag (63, 65) zur Klemmung der Klappprothese (17) in dem Kanal (58) umfasst, um die axiale Verschiebung der äußeren Oberfläche (31) entlang der inneren Oberfläche (57) in mindestens einer Richtung zu blockieren;
**dadurch gekennzeichnet, dass** der Anschlag (63, 65) mindestens eine flexible Verbindung (69) umfasst, die sich quer über den Kanal (58) zwischen zwei Verbindungspunkten (71) erstreckt, die sich auf der inneren Oberfläche (57) befinden und winklig um die Längsachse (Y-Y') mit einem Abstand angeordnet sind.

2. Kit (11; 90; 100) nach Anspruch 1, **dadurch gekennzeichnet, dass** die röhrenförmige Endoprothese (19) zwischen einem komprimierten Zustand und einem erweiterten Zustand ausziehbar ist, wobei die oder jede flexible Verbindung (69) zusammen mit der Endoprothese (19) zwischen einer ausgedehnten Konfiguration, wenn die Endoprothese (19) ihren komprimierten Zustand einnimmt, und einer über den Kanal (58) gespannten Position, wenn die Endoprothese (19) ihren erweiterten Zustand einnimmt, ausziehbar ist.

3. Kit (11; 90; 100) nach einem der Ansprüche 1 oder 2, **dadurch gekennzeichnet**, das die oder jede flexible Verbindung (69) mit Bezug auf die innere Fläche (57) zwischen ihren Verbindungspunkten (71) frei ist.

4. Kit (11; 90; 100) nach einem der vorhergehenden Ansprüche, **dadurch gekennzeichnet, dass** sich die Verbindungspunkte (71) der oder jeder flexiblen Verbindung (69) im Wesentlichen auf einer gleichen Ebene senkrecht zur Längsachse (Y-Y') erstrecken.

5. Kit (11; 90; 100) nach einem der vorhergehenden Ansprüche, **dadurch gekennzeichnet, dass** der oder jeder Anschlag (63, 65) mindestens zwei flexible Verbindungen (69) umfasst, die sich jeweils zwischen zwei Verbindungspunkten (71) erstrecken, wobei die Verhindungspunkte die Scheitelpunkte eines Polygons bilden.

6. Kit (11; 90; 100) nach einem der vorhergehenden Ansprüche, **dadurch gekennzeichnet, dass** die Endoprothese (19) einen proximalen Anschlag (63) und einen distalen Anschlag (65) umfasst, die axial beabstandet sind, wobei jeder Anschlag (63, 65) mindestens eine flexible Verbindung (69) umfasst, die sich quer über den Kanal (58) zwischen zwei Verbindungspunktcn (71) erstrecken, die auf der inneren Oberfläche (57) angebracht sind und winklig um die Längsachse (Y-Y') in einem Abstand angebracht sind.

7. Kit (11; 90; 100) nach Anspruch 6, **dadurch gekennzeichnet, dass** die oder jede flexibie Verbindung (69) mindestens eines Anschlags (63, 65) einen zentralen Durchgang (73) im Kanal (58) begrenzte, wobei das Tragegerüst (29) der Klappenprothese (17) in ihrer zusammengeklappten Position der Anbringung dazu geeignet ist, zwischen den distalen Anschlag (65) und den proximalen Anschlag (63) über den zentralen Durchgang eingeführt zu werden.

8. Kit (11; 90; 100) nach einem der Anspruche 6 oder 7, **dadurch gekennzeichnet, dass** der Abstand, der axial den proximalen Anschlag (63) vom distalen Anschlag (65) trennt, größer oder im Wesentlichen gleich der Länge der äußeren Oberfläche (31) ist, die in Kontakt mit der inneren Oberfläche (57) angebracht ist, genommen entlang der Längsachse (Y-Y').

9. Kit (11; 90; 100) nach einem der vorhergehenden Ansprüche, **dadurch gekennzeichnet, dass** die oder jede flexible Verbindung (69) aus einem Faden gebildet ist.
